# EUROPEAN PATENT SPECIFICATION

(11) **EP 0 640 378 B1**
(45) Date of publication and mention of the grant of the patent: **09.10.1996**
(21) Application number: 94305447.8
(22) Date of filing: 22.07.1994
(51) Int. Cl.: B01D 53/34, C07D 301/19

(54) **Reduction of NOX emissions**
Minderung von NOx-Emissionen
Réduction des émissions de NOx

(30) Priority: 26.07.1993 US 95506
(43) Date of publication of application: 01.03.1995
(73) Proprietor: TEXACO CHEMICAL INC., Houston, Texas 77056 (US)
(72) Inventor: Sliger, Arlen Glenn, Houston, TX (US)
(74) Representative: Green, Mark Charles

(56) References cited:
- EP-A- 0 278 241
- US-A- 4 650 886
- US-A- 5 216 182
- PATENT ABSTRACTS OF JAPAN vol. 14, no. 382 (C-0479) 17 August 1990 & JP-A-02 141 428 (TOKYO TUNGSTEN CO. LTD.) 30 May 1990
- PATENT ABSTRACTS OF JAPAN vol. 5, no. 114 (C-064) 23 July 1981 & JP-A-56 053 794 (TOSHIBA CORP.) 13 May 1982

## Description

This invention relates to a method for reducing the amount of NOX contaminants generated in the practice of a chemical manufacturing process wherein ammonia is formed as a by-product of the chemical process. More particularly, this invention is directed to a method wherein ammonia generated during a chemical manufacturing process is used to reduce the NOX content of flue gas formed during the generation of steam for use in the chemical process. Still more particularly, this invention is directed to a method wherein the feed used in heating a reboiler in a distillation zone for the distillation of chemical reaction products is generated in a steam generator by the combustion of a hydrocarbon fuel with air and wherein ammonia formed during the chemical reaction is used to convert at least a portion of the NOX compounds in the hot combustion gases in the steam generator to molecular nitrogen and water. Even more particularly, this invention is directed to an improvement in a method wherein the steam for use in heating a reboiler in a distillation zone for the distillation of a chemical reaction product is generated in a steam generator comprising a wet steam generation segment and a superheated steam generation segment, wherein hot combustion gases are generated by the burning of a hydrocarbon fuel and air in the steam generator whereby NOX combustion by-products are formed, wherein the hot combustion gases are channelled to provide a flowing stream of hot combustion gases that counter-currently, sequentially flows through the superheated steam generation segment and the wet steam generation segment to a flue and wherein ammonia is formed as one of the chemical reaction products in the chemical process, the improvement comprising the steps of recovering at least a portion of the ammonia in the reaction product and injecting it into the steam generator into the flowing stream of combustion gases at an injection point intermediate to superheated steam generation segment and the wet stream generation segment to thereby convert at least a portion of the NOX combustion product to molecular nitrogen and water vapor.

### 1. Preparation of Molybdenum-containing Catalysts

It is known to prepare molybdenum-containing solutions. One such method is disclosed by Sorgenti in US Patent No. 3573226 wherein molybdenum powder is heated with a stream containing unreacted tertiary butyl hydroperoxide.

Marquis et al. in US Patent No. 4626596, US Patent No. 4650886 and US Patent No, 4703027 disclose methods for the synthesis of molybdenum/alkylene glycol complexes such as molybdenum/ethylene glycol complexes, wherein an ammonium molybdate is reacted with an alkylene glycol in the presence of water to form a solution of a molybdenum/alkylene glycol complex in the alkylene glycol. Mild stripping of the reaction product results in the removal of an off gas comprising ammonia, water vapor and ethylene glycol.

### 2. Manufacture of Propylene Oxide

The manufacture of epoxides such as propylene oxide by the reaction of an olefin such as propylene with a hydroperoxide such as tertiary butyl hydroperoxide is a known process, being described, for example, in Kollar U. S. Patent No. 3,350,422 and U. S. Patent No. 3,351,635.

Marquis et al. in U. S. Patent No. 4,845,251 and U. S. Patent No. 4,891,437 disclose an improvement on the Kollar process wherein a catalyst comprising an ethylene glycol solution of a molybdenum/ethylene glycol complex is used to catalyze the reaction of tertiary butyl hydroperoxide with propylene in a reaction zone to form a reaction product comprising unreacted propylene, propylene oxide, additional tertiary butyl alcohol, unreacted tertiary butyl hydroperoxide and by-products.

### 3. Distillation of Epoxidation Reaction Product

It is known to charge a crude epoxidation reaction product such as one prepared by the reaction of propylene with_ tertiary butyl hydroperoxide in the presence of a molybdenum catalyst to a distillation zone such as one containing a plurality of distillation columns. One such process is disclosed in Schmidt U. S. Patent No. 3,881,996. Another such process is disclosed in Sanderson at al. US patent No. 4810809.

In accordance with one embodiment, the method of the present invention comprises the steps of:
charging ammonia-generating chemical reactants to a chemical preparation zone and reacting them therein to provide a liquid reaction product and an off gas comprising water vapor and ammonia,
charging the liquid reaction product to a distillation zone comprising at least one distillation column having a steam-heated reboiler and separating said liquid reaction products therein into desired distillation fractions,
optionally charging the off gas to a scrubbing zone and counter-currently contacting the off gas therein with water to provide an aqueous extract containing substantially all of the ammonia charged to the scrubbing zone,
charging boiler feed water to a steam generator comprising a combustion segment and a higher temperature superheated steam generation segment, the combustion segment including combustion means for burning a hydrocarbon fuel with air to provide a flowing stream of hot combustion gases containing NOX combustion by-products, thus generating superheated steam,
charging the superheated stream to the steam-heated reboiler in the distillation zone for use in heating the chemical reactants flowing through the reboiler,
and charging the off gas or the aqueous extract to the steam generator and injecting it therein into the flowing stream of hot combustion gases, whereby the ammonia in the off gas or the extract reacts with the NOX compounds in the flowing stream of hot combustion gases to convert the NOX compounds to molecular nitrogen and water.

In a preferred embodiment of the present invention, the invention comprises the steps of:
charging ethylene glycol and an ammonium molybdate to a catalyst preparation zone and reacting them therein to provide a liquid catalyst composition comprising an ethylene glycol solution of an ammonium molybdate/ethylene glycol complex and an off gas comprising water vapor, ammonia and ethylene glycol, charging the liquid catalyst composition together with propylene and a tertiary butyl alcohol solution of tertiary butyl hydroperoxide to an epoxidation reaction zone and catalytically reacting the propylene with the tertiary butyl hydroperoxide therein to provide a liquid reaction product comprising propylene, propylene oxide, tertiary butyl alcohol, unreacted tertiary butyl hydroperoxide and by-products,
charging the liquid reaction product to a distillation zone comprising at least one distillation column having a steam-heated reboiler and separating the liquid reaction product therein into desired fractions by distillation,
charging the off gas to a scrubbing zone and countercurrently contacting the off gas therein to provide an aqueous extract containing substantially all of the ammonia and ethylene glycol charged to the scrubbing zone,
charging boiler feed water to a steam generator comprising a combustion segment, a high temperature super heated steam generation segment and a lower temperature wet steam generation segment, the combustion segment including combustion means for burning a hydrocarbon fuel with air to provide a flowing stream of hot combustion gases containing NOX combustion by-products, the higher temperature super heated steam generating segment being located in the flowing stream of hot combustion gases adjacent said combustion segment and the lower temperature wet steam generation segment being located in the flowing stream of hot combustion gases down stream of and spaced from the higher temperature superheated steam generation segment, the boiler feed water being sequentially passed through the wet steam generation segment and the superheated steam generation segment for indirect heat exchange contact with the flowing stream of hot combustion gases to convert the boiler feed water to superheated steam,
charging the superheated steam to a steam-heated reboiler in the distillation zone to heat the reaction product to be distilled,
and charging the aqueous extract to the steam generator and injecting it therein into the flowing stream of hot combustion gases at a point intermediate the superheated steam generation segment and the wet steam generation segment whereby the ammonia in said extract will react with the NOX compounds in the flowing stream of hot combustion gases to convert the NOX compounds to molecular nitrogen and water.

A preferred embodiment of the present invention will now be described.

### Starting Materials

The starting materials for the present embodiment are a C₃-C₁₂ linear alpha mono olefin, tertiary butyl hydroperoxide, ethylene glycol, an initial ethylene glycol solution of a complex of ethylene glycol with ammonium dimolybdate and a final ethylene glycol solution of a complex of ethylene glycol with ammonium-containing molybdenum compounds derived from a precipitate of solid ammonium-containing molybdenum compounds.

The linear alpha olefins to be used as feedstocks in accordance with the present embodiment are unbranched linear mono olefins containing from 3 to 12 carbon atoms in the molecule including compounds such as propylene, butene-1, pentene-1, hexene-1, octene-1, or dodecane-1. A preferred mono olefin is propylene.

The tertiary butyl hydroperoxide that is used may be a standard industrial grade of tertiary butyl hydroperoxide prepared, for example, by the oxidation of isobutane with oxygen to provide as a reaction product, a solution of tertiary butyl hydroperoxide and tertiary butyl alcohol.

The ethylene glycol is preferably an industrial grade of ethylene glycol that is substantially anhydrous. An industrial grade of ammonium dimolybdate may also be used.

### Preparation of the Initial Ethylene Glycol Solution

The starting materials for the preparation of the initial ethylene glycol solution are ethylene glycol and ammonium dimolybdate. The substantially anhydrous ethylene glycol and ammonium dimolybdate are charged to a catalyst preparation zone in amounts sufficient to provide a molar ratio of about 7 to about 20 moles of ethylene glycol per gram atom of molybdenum contained in the ammonium dimolybdate. The resultant feed mixture is heated at a temperature of about 80° to about 130°C. and more preferably at a temperature of about 90° to about 100°C. at a pressure of 0.1 to 20.8 MPa, and preferably about 0.1 MPa to thereby form a solution of a complex of ethylene glycol with the ammonium dimolybdate in ethylene glycol. The resultant solution is held at a temperature of about 90° to about 120°C. for about 0.5 to about 5 hours sufficient to permit volatilization of volatile reaction components including water and ammonia so as to provide an initial ethylene glycol solution containing about 16 to about 52 wt.% of the complex of ethylene glycol with the ammonium dimolybdate and having a molybdenum content of about 6 to about 20 wt.% and a water concentration of about 0.5 to about 6 wt.%.

### Preparation of the Final Ethylene Glycol Complex

The final ethylene glycol complex is also prepared in the manner described above for the preparation of the initial ethylene glycol solution. However, the source of the molybdenum is not the ammonium dimolybdate specified above but, instead, is a precipitate of a solid ammonium-containing molybdenum compound.

### Epoxidation

In general, the alpha olefin is reacted with tertiary butyl hydroperoxide in solution in tertiary butyl alcohol in the presence of a catalyst mixture composed of the initial ethylene glycol solution and the final ethylene glycol solution by a process of the type disclosed in Kollar U. S. Patent No. 3,351,635, such as a process of the type disclosed in Marquis et al. U. S. Patents No. 4,845,251, No. 4,891,437 or No. 5,107,067.

In particular, the alpha olefin will be used in the form of an anhydrous olefin and the tertiary butyl hydroperoxide will be charged in the form of a solution of about 40 to about 75 wt.% of tertiary butyl hydroperoxide in tertiary butyl alcohol. The reactants will be charged in amounts such that the reaction mixture contains from about 200 to about 600 ppm of the solubilized molybdenum catalyst and such that the molar ratio of alpha olefin to tertiary butyl hydroperoxide is within the range of about 1.05:1 to about 2:1 and, more preferably, within the range of from about 1.05:1 to about 1.8:1, and still more preferably in the range of from about 1.5:1 to about 1.6:1. When the feed materials to the epoxidation reaction zone are charged in the described fashion, the resultant reaction mixture will initially contain more than about 60 wt.% of polar components (tertiary butyl alcohol and tertiary butyl hydroperoxide). The olefin epoxide that is formed during the epoxidation reaction is also a polar material.

The epoxidation reaction may suitably be conducted at a temperature within the range of about 50° to about 180°C., preferably within the range of about 90° to about 140°C., and more preferably within the range from about 100° to about 120°C.

The reaction is suitably conducted at a pressure sufficient to maintain the reactants in liquid phase. Thus, for the higher alpha olefins such as octene-1, the reaction can be conducted at atmospheric pressure. However, a superatmospheric pressure is required for the more volatile lower alpha olefins, such as propylene and butene-1. In this situation, the lower pressure is suitably about 3.5 MPa.

Higher pressures such as pressures within the range of 3.5 to 20.8 MPa may be used if desired. Reaction time may suitably vary from about 0.5 to about 5 hours, and more preferably from about 1.5 to about 2 hours. The reaction may be conducted in a single reaction zone or in a plurality of reaction zones as described, for example, in Marquis et al. U. S. Patent No. 4,891,437.

### Distillation of Reaction Product

The reaction product that is formed when propylene is reacted with tertiary butyl hydroperoxide in solution in tertiary butyl alcohol in the presence of a molybdenum catalyst, as described, will comprise unreacted propylene, propylene oxide, tertiary butyl alcohol, unreacted tertiary butyl hydroperoxide and by-products, and catalyst.

It is conventional to separate the reaction product into a plurality of fractions by distillation in a distillation zone utilizing a plurality of distillation columns. Preferably, the distillation columns that are used are conventional distillation columns comprising a steam-heated reboiler for supplying energy to the distillation column and a reflux condenser to regulate the temperature of the overhead distillate fraction.

In accordance with the conventional practice, the crude reaction product is fractionated sequentially in a series of distillation columns to provide a propylene distillation fraction which is suitably recycled to the chemical reaction zone, a propylene oxide product fraction, a tertiary butyl alcohol product fraction and a heavier distillation fraction, such as a bottoms fraction comprising tertiary butyl alcohol, unreacted tertiary butyl hydroperoxide, molybdenum catalyst, and by-products. Typically, each of the distillation columns will be provided with a conventional reboiler through which a heavy bottoms fraction is circulated for indirect heat exchange contact with a heating medium such as steam or superheated steam to provide the energy required for the distillation.

### Steam Generation

Steam for use in the distillation zone in the reboilers can be generated in a steam generator of any suitable construction such as a flame combustion boiler, a fluidized bed boiler, etc. Within the steam generator, a hydrocarbon fuel is burned with air to provide hot combustion gases having temperatures in excess of 815 °C, such as initial combustion temperatures of about 1315 °C.

The hot combustion gases formed in the combustion segment are channeled so that the stream of flowing hot combustion gases flows through the steam generation portion of the steam generator which may suitably comprise a superheated steam generation segment and a wet steam generation segment. Heat exchange means, such as heating coils, are located at each of the heat exchange segments. Boiler feed water is flowed countercurrent to the direction of flow of the hot combustion gases, initially through the wet steam generation segment and then through the superheated steam generation segment to generate steam which is charged to the distillation zone for use as a heating medium in the distillation column reboilers.

The stream of hot combustion gases may suitably have an initial combustion temperature of 1090 to 1371° C. The hot combustion gases will be cooled in passing through the superheated steam generation segment to temperatures, for example, of from about 980 ° to about 1200°C. The partially cooled combustion gases will then flow through the wet steam generating segment wherein the boiler feed water is converted to wet steam by indirect heat exchange contact. The hot combustion gases are further cooled in the wet steam generation segment to temperatures, for example, of about 180 °C or less.

The thus cooled combustion gases may then be passed to a flue and from thence to a flue stack or to a heat exchange device to recover residual heat contained in the hot combustion gases.

As noted earlier, when the hydrocarbon fuel burns in the presence of air, some of the molecular nitrogen present in the air will be converted to oxygenated by-products, which are collectively referred to as NOX, being actually composed of a variety of oxygenated nitrogen chemical species which, unless removed from the flue gas, will pass to the atmosphere as pollutants.

### NOX Reduction

In accordance with the present invention, the ammonia-containing off gas from the catalyst preparation zone is charged to the steam generator and injected therein at a point intermediate the superheated steam generation segment and the wet steam generation segment so that the ammonia can react with the NOX combustion products by selective noncatalytic reduction and at least partially convert them to water vapor and molecular nitrogen.

In accordance with one embodiment of the present invention, the off gas is injected directly into the flowing stream of hot combustion gases in the steam generator.

In accordance with another embodiment of the present invention, and a preferred embodiment, the ammonia-containing off gas from the catalyst preparation zone is charged to a gas scrubber where it is brought into countercurrent contact with water in order to provide a liquid extract comprising water having substantially all of the ammonia and other water soluble components of the off gas dissolved therein and a tail gas that can be discharged from the process. In accordance with this embodiment of the present invention, the aqueous extract, which contains ammonia, is charged to the steam generator at an injection point intermediate the superheated steam generation segment and the wet steam generation segments. The hot combustion gases will vaporize the water injected at this point liberating the ammonia for reaction with the NOX components of the combustion gases.

The amount of ammonia that is mixed with the off gas should be an amount such that ammonia is not vented to the atmosphere. This can be accomplished by monitoring the concentration of NOX and ammonia in the combustion products. If ammonia is found to be present, an appropriate portion of the off gas can be routed by a separate line to the fuel injection line so that the ammonia contained therein can be combusted together with the hydrocarbon fuel.

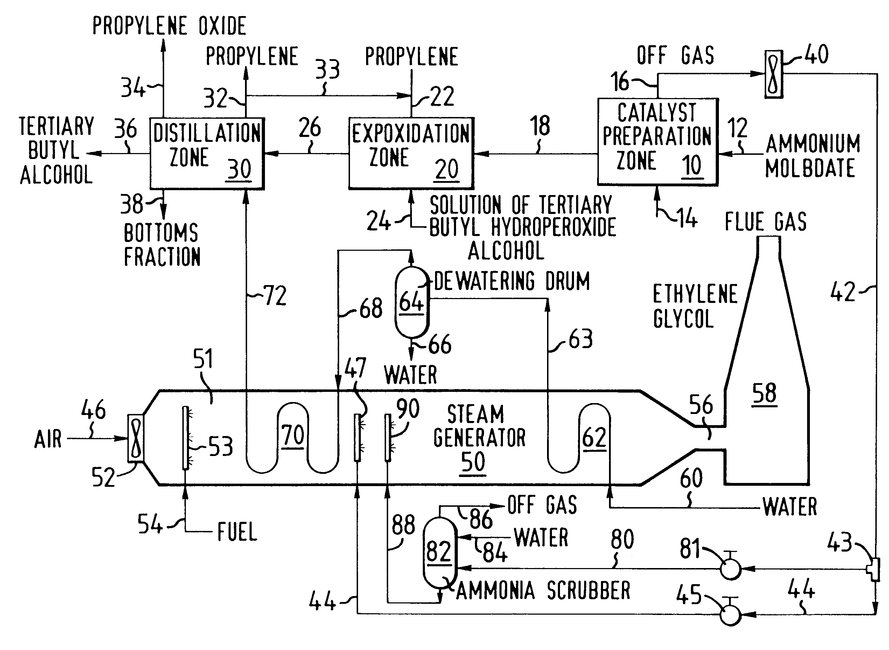

Turning now to the drawing, there is shown a schematic flow sheet illustrating a preferred embodiment of the present invention.

The drawing illustrates an improvement in the PO/MTBE process wherein ammonia generated in the catalyst preparation zone is used to reduce NOX emissions in the flue gas from the steam generator.

A molybdenum catalyst is prepared for the PO/MTBE process in a catalyst preparation zone 10 utilizing a catalyst preparation process such as the process disclosed in Sorgenti U. S. Patent No. 3,573,226, in Marquis et al. U. S. Patents No. 4,626,596, No. 4,650,886 and No. 4,703,027. Thus, an ammonium molybdate is charged to the catalyst preparation zone 10 by a line 12 together with ethylene glycol, which is charged by a line 14. Within the catalyst preparation zone 10 the ammonium molybdate complexes with the ethylene glycol to form an ethylene glycol solution of an ammonium molybdate/ethylene glycol complex that is discharged from the catalyst preparation zone 10 by a line 18. Water and ammonia form as by-products during the complexing reaction and are withdrawn from the catalyst preparation zone 10 by a vent line 16.

The ammonium molybdate/ethylene glycol complex is charged by the line 18 to an epoxidation zone 20 where it is used to catalyze the reaction of propylene with tertiary butyl hydroperoxide by an epoxidation process disclosed in Kollar U. S. Patents No. 3,350,422, No. 3,351,563, etc., by the process disclosed in Marquis et al. U. S. Patents No. 4,845,251, No. 4,891,437. Propylene is charged to the epoxidation zone 20 by a line 22 together with tertiary butyl hydroperoxide which is charged to the epoxidation zone 20 in solution in tertiary butyl alcohol by a line 24. Within the epoxidation zone 20, the propylene reacts with the tertiary butyl hydroperoxide to form propylene oxide, additional tertiary butyl alcohol and by-products.

The epoxidation reaction product formed in the epoxidation zone 20 is discharged therefrom by a line 26 leading to a distillation zone 30 wherein the epoxidation reaction product is separated into distillation fractions by a distillation process such as a distillation process of the type disclosed in Schmidt U. S. Patent No. 3,881,996 or a distillation process of the type disclosed in Sanderson et al. U. S. Patent No. 4,810,809. Thus, for example, the epoxidation reaction product may be separated in the distillation zone 30 into distillation fractions, including a propylene fraction 32 (which is preferably recycled to epoxidation zone 20 by a recycle line 33), a propylene oxide fraction 34, a tertiary butyl alcohol fraction 36 and a bottoms fraction 38.

The water vapor and ammonia in the vent line 16 are charged to a compressor 40 and then by a line 42 from compressor 40 to junction 43 then line 44 to a steam generator 50. The steam generator 50 is suitably used to generate steam to be used as an energy source in the distillation zone 30.

The steam generator 50 may be of any suitable construction known to those skilled in the art. One embodiment of a steam generator is shown in the drawing, but it will be apparent to those skilled in the art that other types of steam generators may be used with satisfactory results.

In the embodiment shown in the drawing, the steam generator 50 comprises a combustion segment 51, a superheated steam generation segment 70, a wet steam generation segment 62 and a flue 58.

Air is charged to the combustion segment 51 of the steam generator 50 by a line 46 leading to an air compressor 52. A hydrocarbon fuel is separately charged to the combustion segment 51 by a line 54 leading to a fuel injection system such as a fuel injection ring 53. The fuel reacts with the air to liberate heat and to form a flowing stream of hot combustion products, including NOX combustion products that flows sequentially from the combustion segment 51, the superheated steam generation segment 70 and the wet steam generation segment 62 to a vent 56 leading to a chimney 58.

Water is charged to the steam generator 50 in a direction of flow countercurrent to the flow of the combustion gases in the steam generator 50, being charged by a line 60 leading to wet steam generation segment 62 where wet steam is generated. The wet steam is discharged from the wet steam generation segment 62 by a line 63 leading to a dewatering drum 64 where water is separated from the steam for discharge through a line 66. The steam is returned to the steam generator 50 by a line 68 leading to superheated steam generation segment 70 where superheated steam is generated for discharge from the superheated steam generation zone 70 by a line 72 leading to the distillation zone 30.

In accordance with the present invention, off gas 16 from the catalyst preparation zone 10, which contains ammonia, water vapor and a minor amount of ethylene glycol is charged to a compressor 40 and then by a line 42 to a junction 43. A line 80 controlled by a valve 81 leads from the junction 43 to an ammonia scrubber 82 of any suitable construction, such as a scrubbing column. The off gas is charged to a feed point adjacent the bottom of the scrubbing column 82 where it is brought into countercurrent contact with scrubbing water charged to the scrubbing column 82 adjacent the top thereof by a water charge line 84. Off gas is discharged by a line 86 and the ammonia-containing wash water is discharged by a line 88 leading to an injection ring 90 located in the flowing stream of hot NOX-containing combustion products at a point between the superheated steam generation segment 70 and the wet stream generation segment 62.

The scrubbing water is vaporized when injected into the flowing stream of hot NOX-containing combustion products and the ammonia contained therein react with the hot NOX-containing combustion products to form water vapor and molecular nitrogen.

Alternately, the valve 81 in the line 80 can be closed and a valve 45 in a line 44 can be opened in order to charge the off gas 16 directly to the flowing stream of hot NOX-containing combustion products by an injection ring 47 so that the ammonia contained in the off gas 16 can react with the hot NOX-containing combustion products to form water vapor and molecular nitrogen.

### SPECIFIC EXAMPLES

The following specific example is given by way of illustration of the present invention and not as a limitation on the scope thereof, as defined by the appended claims.

By way of example, and with reference to the drawing, air may be charged to the steam generator 50 through the line 46 at the rate of about 69200 kg per hour. Fuel is also charged to the combustion zone 51 of the steam generator 50 by way of line 54 at a rate of about 2000 kg per hour, equivalent to a fired duty of about 180 MM btu per hour, sufficient to generate about 77,400 kg per hour of combustion gases.

Boiler feed water is charged to the steam generator 50 through the line 60 to the wet steam generation segment at a rate of about 69,000 kg per hour, to form wet steam which is discharged by a line 63, the wet steam having about the boiling point of water at the pressure of the system.

The wet steam is passed to a dewatering drum 64 where hot water is removed for discharge to a line 66. The wet steam is then charged by line 68 to the superheated steam generation segment 70 at the rate of about 63,500 kg per hour where it is heated to a superheated steam exit generation temperature of about 400° C.

The flue gas after passing through the wet steam generation segment 62 will be at a temperature of about 177°C (350°F) and is discharged to the flue 56 at the rate of about 77,400 kg. per hour.

In accordance with the present invention, an off gas 16 from the catalyst preparation zone 10 is charged to an ammonia scrubber by a line 80 where it is brought into countercurrent contact with water introduced to the ammonia scrubber by a line 84. An extract comprising water having dissolved in it substantially all the ammonia and all the ethylene glycol in the line 80 is discharged by a line 88 leading to the steam generator 50. The off gas is generated in the catalyst preparation zone 10 at the rate of about 5570 kg per hour comprising about 5,440 kg per hour of water, about 120 kg per hour of ammonia and about 5 kg per hour of ethylene glycol.

The extract from the ammonia scrubber is charged by a line 88 to the steam generator for injection through the injection ring 90 at the rate of about 116 kg per hour, constituting an ammonia injection rate of about 2.6 kg per hour.

On being injected into the flowing stream of hot combustion gases, the extract is vaporized and the water is liberated and the ammonia is liberated for reaction with the NOX components in the hot combustion gases to convert them to molecular nitrogen and water vapor.

An aqueous extract introduced into a steam generator in the described manner at the described rate will result in about a 70% reduction of NOX in the boiler flue gas.

## Claims

1. A method for reducing NOX emissions in a chemical reaction process wherein ammonia is formed as a by-product of the chemical reaction and wherein the chemical reaction product is separated into desired fractions by distillation utilizing steam-heated reboilers, wherein the steam for use in the reboilers is generated in a steam generator by the burning of a hydrocarbon fuel with air, whereby NOX combustion by-products are formed, characterized in that at least a portion of the by-product ammonia is injected into the combustion gases inside the steam generator to thereby convert at least a portion of the NOX combustion products to molecular nitrogen and water vapor.

2. A method as claimed in Claim 1, wherein the steam generator comprises a wet steam generation segment, and a superheated steam generation segment, the hot combustion gas flowing counter-currently to boiler feed water sequentially through the superheated steam generation segment and the wet steam generation segment to a flue, characterized in that the by-product ammonia is injected into the combustion gases at an injection point intermediate the superheated steam generation segment and the wet steam generation segment.

3. A method as claimed in Claim 1, wherein the ammonia reaction product is injected into the flowing stream of combustion gases as a gas.

4. A method as claimed in Claim 1, wherein the ammonia reaction product is injected into the flowing stream of combustion gases in solution in liquid water.

5. A method as claimed in Claim 1 or Claim 2, which comprises:
charging ammonia-generating chemical reactants to a chemical preparation zone and reacting them therein to provide a liquid reaction product and an off gas comprising water vapor and ammonia,
charging said liquid reaction product to a distillation zone comprising at least one distillation column having a steam-heated reboiler and separating said liquid reaction products therein into desired distillation fractions,
optionally charging the off gas to a scrubbing zone and counter-currently contacting the off gas therein with water to provide an aqueous extract containing substantially all of the ammonia charged to the scrubbing zone,
charging boiler feed water to a steam generator comprising a combustion segment and a higher temperature superheated steam generation segment, the combustion segment including combustion means for burning a hydrocarbon fuel with air to provide a flowing stream of hot combustion gases containing NOX combustion by-products, thus generating superheated steam,
charging the superheated stream to the steam-heated reboiler in the distillation zone, and
charging the off gas or the aqueous extract to said steam generator and injecting said aqueous extract therein into said flowing stream of hot combustion gases, whereby the ammonia in the off gas or the extract reacts with the NOX compounds in the flowing stream of hot combustion gases to convert the NOX compounds to molecular nitrogen and water.

6. A method as claimed in Claim 1 or Claim 2, characterized in that ethylene glycol and an ammonium molybdate are charged to a catalyst preparation zone and reacted therein to provide a liquid catalyst composition comprising an ethylene glycol solution of an ammonium molybdate/ethylene glycol complex and an off gas comprising water vapor, ammonia and ethylene glycol,
the liquid catalyst composition, propylene and a tertiary butyl alcohol solution of tertiary butyl hydroperoxide are charged to an epoxidation reaction zone and the propylene reacted with the tertiary butyl hydroperoxide therein to provide a liquid reaction product comprising propylene, propylene oxide, tertiary butyl alcohol, unreacted tertiary butyl hydroperoxide and by-products,
the liquid reaction product is charged to a distillation zone, and
the off gas is charged to a scrubbing zone to provide an aqueous extract containing substantially all of the ammonia and ethylene glycol charged to said scrubbing zone.

## Patentansprüche

1. Ein Verfahren Zur Minderung von NO_{X}-Emissionen in einem chemischen Reaktionsverfahren, bei dem Ammoniak als ein Nebenprodukt der chemischen Reaktion gebildet wird und bei dem das chemische Reaktionsprodukt durch Destillation unter Verwendung dampfbeheizter Blasen in gewünschte Fraktionen aufgetrennt wird, wobei der Dampf zur Verwendung in den Blasen in einem Dampferzeuger durch das Verbrennen eines Kohlenwasserstoffbrennstoffes mit Luft erzeugt wird, wodurch NO_{X}-Verbrennungsnebenprodukte gebildet werden, dadurch gekennzeichnet, daß wenigstens ein Teil des Nebenproduktes Ammoniak innerhalb des Dampferzeugers in die Verbrennungsgase eingespritzt wird, um dadurch Wenigstens einen Teil der NO_{X}-Verbrennungsprodukte zu molekularem Stickstoff und Wasserdampf umzuwandeln.

2. Ein Verfahren nach Anspruch 1, wobei der Dampferzeuger ein Segment zur Erzeugung von Naßdampf und ein Segment zur Erzeugung von überhitztem Dampf umfaßt, wobei das heiße Verbrennungsgas im Gegenstrom zum Kesselspeisewasser nacheinander durch das Segment zur Erzeugung von überhitztem Dampf und das Segment zur Erzeugung von Naßdampf zu einem Zug strömt, dadurch gekennzeichnet, daß das Nebenprodukt Ammoniak in die Verbrennungsgase an einem Einspritzpunkt zwischen dem Segment zur Erzeugung von überhitztem Dampf und dem Segment zur Erzeugung von Naßdampf eingespritzt wird.

3. Ein Verfahren nach Anspruch 1, wobei das Ammoniak-Reaktionsprodukt in den Fließstrom der Verbrennungsgase als ein Gas eingespritzt wird.

4. Ein Verfahren nach Anspruch 1, wobei das Ammoniak-Reaktionsprodukt in den Fließstrom der Verbrennungsgase in Lösung in flüssigem Wasser eingespritzt wird.

5. Ein Verfahren nach Anspruch 1 oder Anspruch 2, welches umfaßt:
daß Ammoniak-erzeugende chemische Reaktanten in eine chemische Herstellungszone eingebracht und sie darin zur Reaktion gebracht werden, um ein flüssiges Reaktionsprodukt und ein Abgas bereitzustellen, das Wasserdampf und Ammoniak umfaßt,
daß besagtes flüssiges Reaktionsprodukt in eine Destillationszone eingebracht wird, die wenigstens eine Destillationskolonne mit einer dampfbeheizten Blase umfaßt, und besagte flüssigen Reaktionsprodukte darin in gewünschte Destillationsfraktionen aufgetrennt werden,
daß fakultativ das Abgas in eine Gaswaschzone eingebracht und das Abgas darin im Gegenstrom mit Wasser in Kontakt gebracht wird, um einen wäßrigen Extrakt bereitzustellen, der im wesentlichen das gesamte Ammoniak, das der Gaswaschzone zugeführt wird, enthält,
daß Kesselspeisewasser in einen Dampferzeuger eingebracht wird, der ein Verbrennungssegment und ein Segment zur Erzeugung von überhitztem Dampf mit höherer Temperatur umfaßt, wobei das Verbrennungssegment Verbrennungsmittel zur Verbrennung eines Kohlenwasserstoffbrennstoffes mit Luft einschließt, um einen Fließstrom von heißen Verbrennungsgasen bereitzustellen, die NO_{X}-Verbrennungsnebenprodukte enthalten, wodurch überhitzter Dampf erzeugt wird,
daß der überhitzte Strom in die dampfbeheizte Blase in der Destillationszone eingebracht wird und
daß das Abgas oder der wäßrige Extrakt in besagten Dampferzeuger eingebracht wird und besagter wäßrige Extrakt darin in besagten Fließstrom aus heißen Verbrennungsgasen eingespritzt wird, wodurch das Ammoniak im Abgas oder der Extrakt mit den NO_{X}-Verbindungen im Fließstrom aus heißen Verbrennungsgasen reagiert, um die NO_{X}-Verbindungen zu molekularem Stickstoff und Wasser umzuwandeln.

6. Ein Verfahren nach Anspruch 1 oder Anspruch 2, dadurch gekennzeichnet, daß Ethylenglykol und ein Ammoniummolybdat in eine Katalysatorherstellungszone eingebracht und darin zur Reaktion gebracht werden, um eine flüssige Katalysatorzusammensetzung, die eine Ethylenglykollösung eines Ammoniummolybdat/Ethylenglykol-Komplexes umfaßt, und ein Abgas bereitzustellen, das Wasserdampf, Ammoniak und Ethylenglykol umfaßt,
daß die flüssige Katalysatorzusammensetzung, Propylen und eine tert.-Butylalkohollösung von tert.-Butylhydroperoxid in eine Epoxidationsreaktionszone eingebracht und das Propylen mit dem tert.-Butylhydroperoxid darin zur Reaktion gebracht wird, um ein flüssiges Reaktionsprodukt bereitzustellen, das Propylen, Propylenoxid, tert.-Butylalkohol, nicht-umgesetztes tert.-Butylhydroperoxid und Nebenprodukte umfaßt,
daß das flüssige Reaktionsprodukt in eine Destillationszone eingebracht wird und
daß das Abgas in eine Gaswaschzone eingebracht wird, um einen wäßrigen Extrakt bereitzustellen, der im wesentlichen das gesamte Ammoniak und Ethylenglykol enthält, das besagter Gaswaschzone zugeführt wird.

## Revendications

1. Procédé pour réduire les émissions de NOₓ dans un procédé de réaction chimique dans lequel de l'ammoniac est formé en tant que sous-produit de la réaction chimique et dans lequel le produit de la réaction chimique est séparé en fractions souhaitées par distillation, en utilisant des rebouilleurs chauffés à la vapeur, la vapeur pour l'utilisation dans les rebouilleurs étant produite dans un générateur de vapeur par la combustion d'un carburant hydrocarboné avec de l'air, par laquelle des sous-produits de combustion NOₓ sont formés, caractérisé en ce qu'au moins une partie de l'ammoniac sous-produit est injecté dans les gaz de combustion dans le générateur de vapeur pour convertir ainsi au moins une partie des produits de combustion NOₓ en azote moléculaire et vapeur d'eau.

2. Procédé selon la revendication 1, dans lequel le générateur de vapeur comprend un segment produisant de la vapeur d'eau humide et un segment produisant de la vapeur d'eau surchauffée, le gaz de combustion chaud s'écoulant à contre-courant de l'eau d'alimentation de chaudière, successivement au travers du segment produisant de la vapeur d'eau surchauffée et du segment produisant de la vapeur d'eau humide vers un tuyau de cheminée, caractérisé en ce que l'ammoniac sous-produit est injecté dans les gaz de combustion à un point d'injection intermédiaire entre le segment produisant de la vapeur d'eau surchauffée et le segment produisant de la vapeur d'eau humide.

3. Procédé selon la revendication 1, dans lequel le produit de réaction ammoniac est injecté, sous la forme d'un gaz, dans le courant de gaz de combustion s'écoulant.

4. Procédé selon la revendication 1, dans lequel le produit de réaction ammoniac est injecté, dans le courant de gaz de combustion s'écoulant en solution dans de l'eau liquide.

5. Procédé selon la revendication 1 ou la revendication 2, qui comprend les étapes consistant :
à charger les réactifs chimiques produisant de l'ammoniac dans une zone de préparation chimique et à les y faire réagir pour fournir un produit de réaction liquide et un effluent gazeux comprenant de la vapeur d'eau et de l'ammoniac,
à charger ledit produit de réaction liquide dans une zone de distillation comprenant au moins une colonne de distillation ayant un rebouilleur chauffé à la vapeur, et à y séparer lesdits produits de réaction liquides en fractions de distillation souhaitées,
facultativement, à charger l'effluent gazeux dans une zone de lavage et à y mettre en contact l'effluent gazeux, à contre-courant avec de l'eau pour fournir un extrait aqueux contenant substantiellement tout l'ammoniac chargé à la zone de lavage,
à charger de l'eau d'alimentation de chaudière dans un générateur de vapeur comprenant un segment de combustion et un segment de plus haute température produisant de la vapeur surchauffée, le segment de combustion incluant des moyens de combustion pour brûler un combustible hydrocarboné avec de l'air pour fournir un courant de gaz de combustion chauds s'écoulant contenant des sous-produits de combustion NOₓ, produisant ainsi de la vapeur surchauffée, et
à charger la vapeur surchauffée dans le rebouilleur chauffé à la vapeur dans la zone de distillation, et
à charger l'effluent gazeux ou l'extrait aqueux dans ledit générateur de vapeur et à y injecter ledit extrait aqueux, dans ledit courant de gaz de combustion chauds s'écoulant, de sorte que l'ammoniac dans l'effluent gazeux ou l'extrait réagit avec les composés NOₓ dans le courant de gaz de combustion chauds s'écoulant, pour convertir les composés NOₓ en azote moléculaire et eau.

6. Procédé selon la revendication 1 ou la revendication 2, caractérisé en ce que de l'éthylèneglycol et un molybdate d'ammonium sont chargés dans une zone de préparation de catalyseur et y réagissent pour fournir une composition de catalyseur liquide comprenant une solution, dans l'éthylèneglycol, d'un complexe molybdate d'ammonium/éthylèneglycol, et un effluent gazeux comprenant de la vapeur d'eau, de l'ammoniac et de l'éthylèneglycol,
la composition de catalyseur liquide, du propylène et une solution, dans de l'alcool tertiobutylique, d'hydroperoxyde de tertiobutyle sont chargés dans une zone de réaction d'époxydation, et on y fait réagir le propylène avec l'hydroperoxyde de tertiobutyle pour fournir un produit de réaction liquide comprenant du propylène, de l'oxyde de propylène, de l'alcool tertiobutylique, de l'hydroperoxyde de tertiobutyle n'ayant pas réagi et des sous-produits,
le produit de réaction liquide est chargé dans une zone de distillation, et
l'effluent gazeux est chargé dans une zone de lavage pour fournir un extrait aqueux contenant substantiellement tout l'ammoniac et l'éthylèneglycol chargé dans ladite zone de lavage.
